# EUROPEAN PATENT APPLICATION

(11) **EP 3 357 572 A1**
(43) Date of publication of application: **08.08.2018**
(21) Application number: 16851076.6
(22) Date of filing: 08.09.2016
(51) Int. Cl.: B01J 19/12, A61L 9/20, C02F 1/32

(54) **IRRADIATION DEVICE AND FLUID STERILIZATION METHOD**

(30) Priority: 29.09.2015 JP 2015191564
(71) Applicant: Nikkiso Co., Ltd., Shibuya-ku, Tokyo 150-6022 (JP)
(72) Inventor: OCHI Tetsumi, Hakusan-shi Ishikawa 924-0004 (JP); KONAGAYOSHI Hidenori, Kariya-city Aichi 448-8661 (JP); TORII Nobuhiro, Kariya-city Aichi 448-8661 (JP); KIUCHI Hiroki, Kariya-city Aichi 448-8661 (JP)
(74) Representative: Copsey, Timothy Graham
(86) International application number: PCT/JP2016/076421
(87) International publication number: WO 2017/056902

(57) **Abstract**

An irradiation apparatus 10 is provided with: a straight pipe 20 that is formed of polytetrafluoroethylene (PTFE); and a light source 40 that is arranged at an end portion (first end portion 22) of the straight pipe 20 and that irradiates the inside of the straight pipe 20 with ultraviolet light. The light source 40 has a light emitting device 42 that emits ultraviolet light and an adjustment mechanism 50 that adjusts the direction of the ultraviolet light such that the ultraviolet light from the light emitting device 42 becomes incident on the internal wall surface 20a of the straight pipe 20 at an incident angle θ of 75 degrees or more.

## Description

### [TECHNICAL FIELD]

The present invention relates to an irradiation apparatus and a fluid sterilization method and particularly to a technology for sterilizing a fluid by irradiation with ultraviolet light.

### [BACKGROUND ART]

Ultraviolet light is known to have sterilization capability, and apparatuses are used that radiate ultraviolet light for sterilization treatment performed at medical sites, food processing sites, etc. Also, apparatuses are used that sterilize, by irradiating a fluid such as water with ultraviolet light, the fluid in a continuous manner. Such apparatuses include, for example, apparatuses where an ultraviolet LED is arranged on the internal wall of a pipe end of a flow passage formed with a straight metal pipe (see, for example, Patent Document 1).

[Patent Document 1] Japanese Patent Application Publication No. 2011-16074

### [DISCLOSURE OF THE INVENTION]

### [PROBLEM TO BE SOLVED BY THE INVENTION]

In order to efficiently irradiate, with ultraviolet light, a fluid flowing inside a straight-pipe shaped flow passage, it is desirable to achieve a structure where ultraviolet light reflectivity at an internal wall surface of the flow passage is high. Also, it is desirable to form the internal wall surface of the flow passage with a material that is unlikely to be corroded by the fluid flowing inside the flow passage.

In this background, one of exemplary purposes of the present invention is to provide an irradiation apparatus with increased efficiency for irradiating the inside of a straight pipe with ultraviolet light.

### [MEANS TO SOLVE THE PROBLEM]

An irradiation apparatus according to one embodiment of the present invention is provided with: a straight pipe that is formed of polytetrafluoroethylene (PTFE); and a light source that is arranged at an end portion of the straight pipe and that irradiates the inside of the straight pipe with ultraviolet light. The light source has a light emitting device that emits ultraviolet light and an adjustment mechanism that adjusts the direction of the ultraviolet light such that the ultraviolet light from the light emitting device becomes incident on the internal wall surface of the straight pipe at an incident angle of 75 degrees or more.

According to this embodiment, by allowing ultraviolet light to become incident on the internal wall surface of PTFE at an incident angle of 75 degrees or more, the reflectivity of the ultraviolet light at the internal wall surface can be increased so that the ultraviolet light can be guided efficiently in the longitudinal direction of the straight pipe. Based on the knowledge of the inventors, it is understood that, when ultraviolet light is allowed to become incident on the surface of PTFE at an incident angle of 75 degrees or more, a specular reflection component becomes larger than a diffuse reflection component and almost all the ultraviolet light is reflected. Therefore, according to the present embodiment, effects in which ultraviolet light is transmitted through the internal wall surface of PTFE and in which ultraviolet light goes back to the side of the light source due to diffuse reflection becoming predominant can be reduced, and the intensity of ultraviolet light inside the straight pipe can be maintained at a high level throughout the longitudinal direction of the straight pipe. This allows for an increase in the efficiency of the irradiation inside the straight pipe with ultraviolet light. Further, since PTFE, which is a chemically stable fluororesin, is used as a straight pipe that forms a flow passage, the durability of the apparatus can be improved.

The adjustment mechanism may adjust the direction of the ultraviolet light such that the light distribution angle of the ultraviolet light irradiating the inside of the straight pipe is 30 degrees or less.

The length of the straight pipe may be three or more times larger than the diameter of the straight pipe.

The light source may irradiate a fluid flowing inside the straight pipe with ultraviolet light to perform sterilization treatment on the fluid.

Another embodiment of the present invention relates to a fluid sterilization method. This method includes irradiating a fluid flowing inside a straight pipe formed of polytetrafluoroethylene (PTFE) with ultraviolet light to perform sterilization treatment on the fluid. The ultraviolet light is irradiated in a direction where the ultraviolet light becomes incident on the internal wall surface of the straight pipe at an incident angle of 75 degrees or more.

According to this embodiment, by allowing ultraviolet light to become incident on the internal wall surface of PTFE at an incident angle of 75 degrees or more, the reflectivity of the ultraviolet light at the internal wall surface can be increased so that the ultraviolet light can be guided efficiently in the longitudinal direction of the straight pipe. This allows for an increase in the efficiency of the irradiation inside the straight pipe with ultraviolet light and thus allows the efficiency of sterilizing the fluid flowing inside the straight pipe to be improved. Further, by using PTFE, which is a chemically stable fluororesin, as a straight pipe that forms a flow passage, the durability of the straight pipe can be improved.

### [ADVANTAGE OF THE INVENTION]

According to the present invention, sterilization capability can be improved by increasing efficiency for irradiating the inside of a straight pipe with ultraviolet light.

### [BRIEF DESCRIPTION OF THE DRAWINGS]

Fig. 1 is a cross-sectional view schematically showing the configuration of an irradiation apparatus according to an embodiment;
Fig. 2 is a diagram schematically showing the measurement of reflection characteristics of a PTFE plate;
Fig. 3 is a graph showing the reflection characteristics of the PTFE plate;
Fig. 4 is a cross-sectional view schematically showing the configuration of an irradiation apparatus according to a comparative example;
Fig. 5 is a graph showing the ultraviolet light intensity of the inside of a straight pipe; and
Fig. 6 is a cross-sectional view schematically showing the configuration of a light source according to an exemplary variation.

### [MODE FOR CARRYING OUT THE INVENTION]

Hereinafter, an embodiment for carrying out the present invention will be described in detail with reference to the accompanying drawing. In the explanation, like numerals represent like constituting elements, and duplicative explanations will be omitted appropriately.

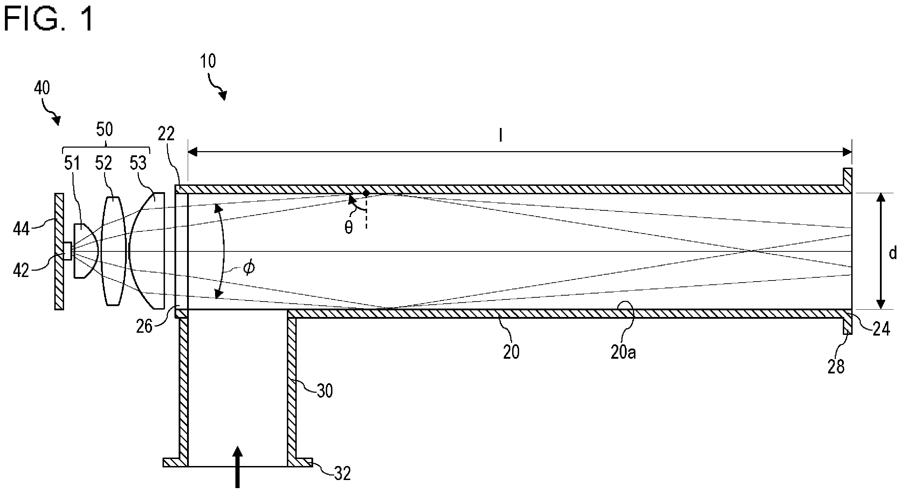

Fig. 1 is a diagram schematically showing the configuration of an irradiation apparatus 10 according to an embodiment. The irradiation apparatus 10 is provided with a straight pipe 20, a connecting pipe 30, and a light source 40. The light source 40 is arranged at an end portion (first end portion 22) of the straight pipe 20 and that irradiates the inside of the straight pipe 20 with ultraviolet light. The irradiation apparatus 10 is used, for example, for irradiating a fluid such as water flowing inside the straight pipe 20 with ultraviolet light to perform sterilization treatment.

The straight pipe 20 has a first end portion 22, a second end portion 24, a window portion 26, and a second flange 28. The straight pipe 20 extends in a longitudinal direction toward the second end portion 24 from the first end portion 22 and has a length 1, which is three or more times larger than an inner diameter (diameter) d. The window portion 26 for transmitting ultraviolet light from the light source 40 is provided at the first end portion 22. The window portion 26 is formed of a member having high ultraviolet light transmittance such as quartz (SiO₂), sapphire (Al₂O₃), non-crystalline fluorine-based resin, etc. A flange (second flange 28) for connecting the straight pipe 20 to another pipe or the like is provided at the second end portion 24.

Further, at the first end portion 22, the connecting pipe 30 extending in a direction that intersects with or is perpendicular to the longitudinal direction of the straight pipe 20 is attached. A flange (first flange 32) is provided at one end of the connecting pipe 30, and the straight pipe 20 is attached to the other end. The straight pipe 20 and the connecting pipe 30 form an L-shaped flow passage. For example, a fluid flowing in from the first flange 32 flows out from the second flange 28 through the connecting pipe 30 and the straight pipe 20. A fluid may flow in the opposite direction, and a structure may be employed where a fluid flowing in from the second flange 28 flows out from the first flange 32.

The straight pipe 20 and the connecting pipe 30 are formed of polytetrafluoroethylene (PTFE), which is a perfluorinated resin. PTFE is a chemically stable material and is a material excellent in durability, heat resistance, and chemical resistance. Also, PTFE is a material with high ultraviolet light reflectivity. Therefore, the straight pipe 20 can reflect, at an internal wall surface 20a, ultraviolet light emitted by the light source 40 and propagate the ultraviolet light in the longitudinal direction of the straight pipe 20.

The straight pipe 20 and the connecting pipe 30 do not need to be entirely formed of PTFE, as long as at least the internal wall surfaces thereof, which form the flow passage and come into contact with a fluid, are formed of PTFE. For example, the straight pipe 20 and the connecting pipe 30 may be formed by attaching a liner made of PTFE to the internal surfaces of the pipes formed of other resin materials or metal materials.

The light source 40 includes a light emitting device 42, a substrate 44, and an adjustment mechanism 50. The light emitting device 42 is an LED (Light Emitting Diode) that emits ultraviolet light with a center wavelength or peak wavelength that is included in a range of about 200 nm to 350 nm. The light emitting device 42 preferably emits ultraviolet light of around 260 nm to 270 nm, which is a wavelength for high sterilization efficiency. As such an ultraviolet light LED, for example, those in which aluminum gallium nitride (AlGaN) is used are known.

The light emitting device 42 is mounted on the substrate 44 so as to face the adjustment mechanism 50. The substrate 44 is formed of a material with high thermal conductivity, and, for example, copper (Cu), aluminum (Al), or the like is used as a base material. Heat generated by the light emitting device 42 is dissipated via the substrate 44.

The light emitting device 42 is an LED with a wide light distribution angle having a directivity angle or a light distribution angle of 60 degrees or more, 90 degrees or more, or 120 degrees or more. Such a light emitting device 42 includes an LED of a surface mount type (SMD: surface mount device) with high output intensity. Ultraviolet light emitted by the light emitting device 42 enters the adjustment mechanism 50, and the direction of the ultraviolet light is adjusted by the adjustment mechanism 50.

The adjustment mechanism 50 adjusts the direction of the ultraviolet light such that the ultraviolet light from the light emitting device 42 becomes incident on the internal wall surface 20a of the straight pipe 20 at an incident angle θ of 75 degrees or more. The adjustment mechanism 50 adjusts the light distribution angle of the ultraviolet light emitted by the light emitting device 42 such that the light distribution angle ϕ of the ultraviolet light output from the adjustment mechanism 50 is 30 degrees or less. Further, the adjustment mechanism 50 is arranged such that the optical axis direction of the ultraviolet light output from the adjustment mechanism 50 is the longitudinal direction of the straight pipe 20. Setting the incident angle θ of the ultraviolet light becoming incident on the internal wall surface 20a to be 75 degrees or more by using such an adjustment mechanism 50 allows the reflectivity of the ultraviolet light at the internal wall surface 20a to be increased and thus allows high-intensity ultraviolet light to be propagated throughout the longitudinal direction of the straight pipe 20.

The adjustment mechanism 50 has a first lens 51, a second lens 52, and a third lens 53. Each of the lenses is formed of a quartz glass, which has high ultraviolet light transmittance. The ultraviolet light emitted by the light emitting device 42 is transmitted through the first lens 51, the second lens 52, the third lens 53, and the window portion 26 in this order and is irradiated to the inside of the straight pipe 20. As shown in the figure, the first lens 51 is a plano-convex lens, the second lens 52 is a biconvex lens, and the third lens 53 is a plano-convex lens. The adjustment mechanism 50 may be composed of two or less lenses or may be composed of four or more lenses. The lenses the adjustment mechanism 50 has may be lenses having shapes shown in the figure or lenses having different shapes.

An explanation will be given now regarding the reflection characteristics of PTFE that forms the internal wall surface 20a of the straight pipe 20. Although PTFE is a resin material that is versatilely used for various purposes, quantitative reflection characteristics of PTFE with regard to deep ultraviolet light is not known much. Although a feature of PTFE has been suggested where an angular component of reflected light can vary depending on the angle of incident light due to the nature of PTFE being a resin material, the detailed reflection characteristics of PTFE when irradiated with deep ultraviolet light are not known much. The inventors of the present invention consider improving the optical characteristics of the irradiation apparatus 10 in which PTFE is used by measuring the reflection characteristics of PTFE with regard to deep ultraviolet light and taking good advantage of the characteristics.

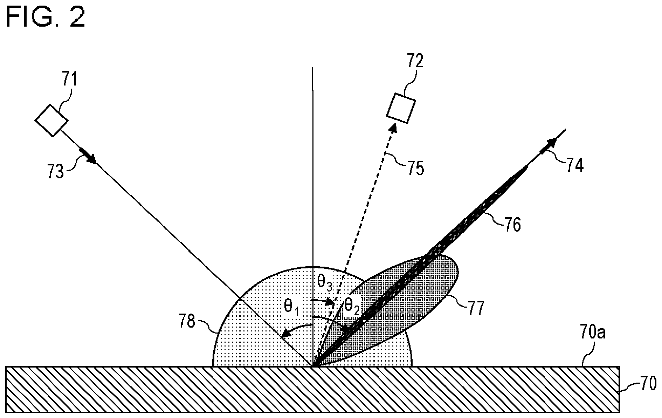

Fig. 2 is a diagram schematically showing the measurement of the reflection characteristics of a PTFE plate 70. Incident light 73 from a light source 71, which emits ultraviolet light, is transmitted, reflected, or scattered at a surface 70a of the PTFE plate 70. In general, reflection at an object surface is known to be able to be classified into three components: a first specular reflection (specular spike) component 76; a second specular reflection (specular lobe) component 77; and a diffuse reflection (diffuse lobe) component 78. The inventors of the present invention obtained these three components for the PTFE plate 70 by measuring, using a measuring instrument 72, the intensity of reflected light 74 having a reflection angle θ₂ that is the same as an incident angle θ₁ of the incident light 73, scattered light 75 having a scattering angle θ₃ that is the different from the incident angle θ₁, or the like.

Of the three components shown in Fig. 2, the first specular reflection component 76 is very strong reflected light that is reflected at the surface 70a and is radiated in an extremely narrow angle range in a specular reflection direction θ₂. The second specular reflection component 77 is strong reflected light that is reflected at the surface 70a and is radiated in a spreading manner while having the specular reflection direction θ₂ as an approximate center. The diffuse reflection component 78 is reflected light that is radiated to inside the PTFE plate 70 with repeating scattering and is radiated isotropically from the surface 70a without depending on the scattering angle θ₃.

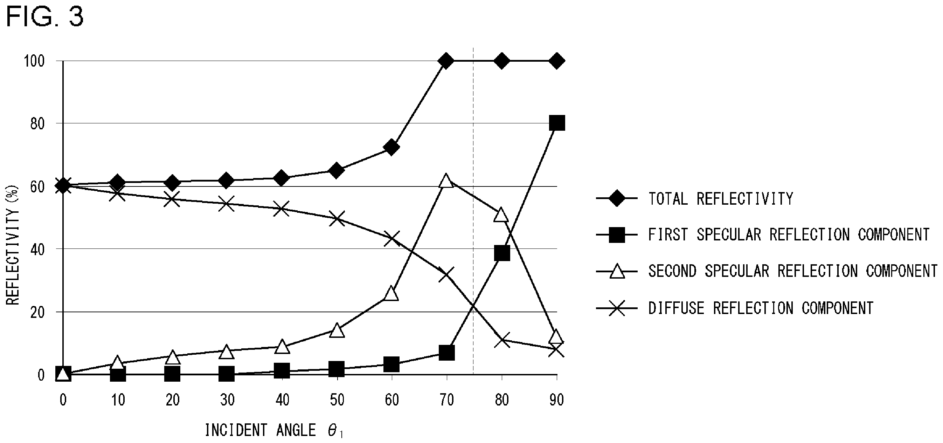

Fig. 3 is a graph showing the reflection characteristics of the PTFE plate 70 and shows the respective reflectivities of the first specular reflection component 76, the second specular reflection component 77, and the diffuse reflection component 78 with respect to the incident angle θ₁ of the incident light 73 and a total reflectivity obtained by combining these respective reflectivities of the three components. As the light source 71, an LED that emits ultraviolet light having a wavelength λ of 280 nm was used. As shown in the figure, it can be found that, when the incident angle θ₁ is in a range of 0 to 60 degrees, reflected light is mostly composed of the diffuse reflection component 78 and the ratios of the first specular reflection component 76 and the diffuse reflection component 78 are small. It can be also found that the total reflectivity is 80 percent or less and that 20 percent or more of the incident light 73 is transmitted without getting reflected.

On the other hand, when the incident angle θ₁ exceeds 60 degrees and becomes 70 degrees or more, the ratio of the diffuse reflection component 78 becomes decreased and the ratio of the second specular reflection component 77 becomes increased. Further, the total reflectivity becomes 100 percent, and the influence of a loss caused due to the transmission of the incident light 73 becomes diminished. Further, it can be found that, when the incident angle θ₁ becomes 75 degrees or more, the diffuse reflection component 78 accounts for around 20 percent or 20 percent or less and around 80 percent or 80 percent or more of the incident light 73 is reflected in the specular reflection direction without any loss. Based on this measurement result, the inventors of the present invention consider that setting the incident angle θ of the ultraviolet light becoming incident on the internal wall surface 20a of PTFE to be 75 degrees or more allows a large portion of the reflected light to be propagated in the longitudinal direction of the straight pipe 20, thus allowing high-intensity ultraviolet light to be guided farther.

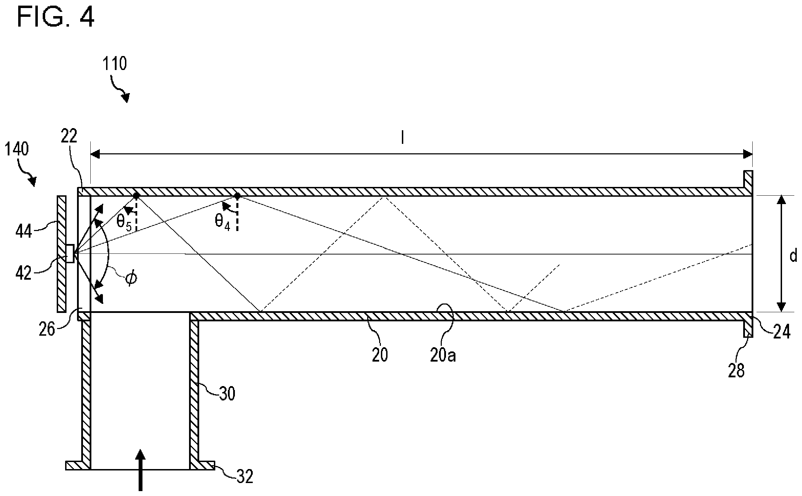

Then, an explanation will be given regarding the effects of the irradiation apparatus 10 in reference to a comparative example. Fig. 4 is a cross-sectional view schematically showing the configuration of an irradiation apparatus 110 according to the comparative example. The irradiation apparatus 110 is different from the above-stated embodiment in that a structure is employed where the above-stated adjustment mechanism 50 is not included in the light source 140 and ultraviolet light emitted by the light emitting device 42 directly irradiates the inside of the straight pipe 20.

The light source 140 irradiates the inside of the straight pipe 20 with the ultraviolet light directly from the light emitting device 42 and has a light distribution angle ϕ that is larger than that of the light source 40 according to the above-stated embodiment. Therefore, as shown in the figure, a portion of the ultraviolet light emitted from the light source 140 becomes incident on the internal wall surface 20a of the straight pipe 20 at incident angles θ₄ and θ₅ that are smaller than 75 degrees. Specular reflection components of ultraviolet light having such incident angles θ₄ and θ₅ are small, and a large portion of the ultraviolet light that is incident is scattered isotropically by diffuse reflection or transmitted through the internal wall of the straight pipe 20. In that case, components that become reflected in the longitudinal direction of the straight pipe 20 become extremely small, and it becomes difficult to guide high-intensity ultraviolet light throughout the longitudinal direction of the straight pipe 20.

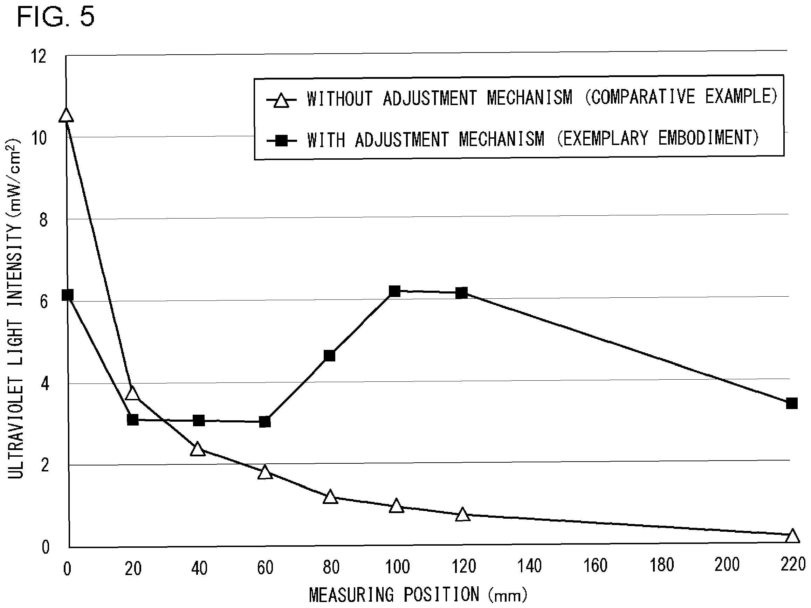

Fig. 5 is a graph showing the ultraviolet light intensity of the inside of the straight pipe 20 and shows ultraviolet light intensity in the irradiation apparatus 10 according to the embodiment that has the adjustment mechanism 50 and ultraviolet light intensity in the irradiation apparatus 110 according to the comparative example that does not have an adjustment mechanism 50. Fig. 5 shows ultraviolet light intensity at the center position of the straight pipe 20 when the length 1 of the straight pipe 20 is changed while the inner diameter of the straight pipe 20 is set as follows: d = 20 mm.

As shown in the figure, in a measurement result according to the comparative example without an adjustment mechanism 50, it can be found that the ultraviolet light intensity becomes gradually reduced in accordance with the length 1 of the straight pipe 20. It is considered that this is due to a decrease in components heading in the longitudinal direction of the straight pipe 20 that occurs every time reflection occurs at the internal wall surface 20a of the straight pipe 20. On the other hand, in a measurement result according to the embodiment with the adjustment mechanism 50, it can be found that a predetermined intensity or more is maintained even when the length 1 of the straight pipe 20 becomes longer. In particular, in a range where the length 1 of the straight pipe 20 is 60 mm or more, which is three or more times the inner diameter d (20 mm), it can be found that the ultraviolet light intensity in the embodiment is significantly larger than that in the comparative example.

In the above configuration, by irradiating, with ultraviolet light, a fluid flowing inside the straight pipe 20 formed of PTFE, the irradiation apparatus 10 performs sterilization treatment on the fluid. Ultraviolet light is irradiated in a direction where the ultraviolet light becomes incident on the internal wall surface 20a of the straight pipe 20 at an incident angle θ of 75 degrees or more. In the ultraviolet light that becomes incident on the internal wall surface 20a at an incident angle θ of 75 degrees or more, almost all the components are reflected at the internal wall surface 20a, and most of the components are specularly reflected and travel in the longitudinal direction of the straight pipe 20. Therefore, the fluid flowing along the longitudinal direction of the straight pipe 20 can be irradiated with high-intensity ultraviolet light throughout the longitudinal direction. This allows a range and time for which high-intensity ultraviolet light acts on a fluid to become longer, and sterilization action on the fluid can be improved.

According to the present embodiment, since PTFE is used for the internal wall of the straight pipe 20, the reliability of the straight pipe 20 can be increased compared to a case where a metal material such as aluminum (Al) is used. Although aluminum is known as a material with high ultraviolet light reflectivity, when water is used as the fluid, electric corrosion or corrosion may occur due to aluminum becoming into contact with water, lowering the ultraviolet light reflectivity or leading to a hygiene-related concern. On the other hand, according to the present embodiment, since chemically stable PTFE is used, such a concern can be prevented. Therefore, according to the present embodiment, the reliability of the irradiation apparatus 10 can be improved as well as improving the ultraviolet light irradiation efficiency.

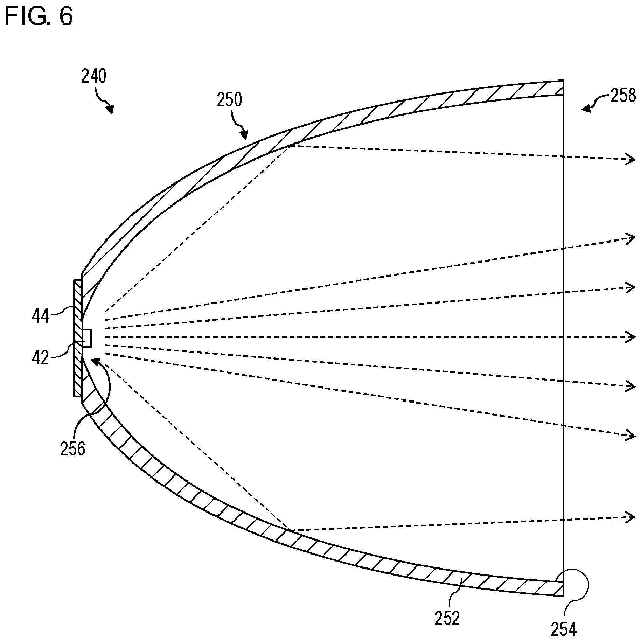

Fig. 6 is a cross-sectional view schematically showing the configuration of a light source 240 according to an exemplary variation. The light source 240 includes a light emitting device 42, a substrate 44, and an adjustment mechanism 250. The adjustment mechanism 250 according to the comparative example is different from the above-stated embodiment in that the direction of ultraviolet light from the light emitting device 42 is adjusted by a reflection-type structure. An explanation will be given in the following mainly regarding differences.

The adjustment mechanism 250 has a reflector 252. The reflector 252 is formed of a metal material or a resin material, and a reflection surface 254 is formed of a material having high ultraviolet light reflectivity. The reflector 252 is formed of, for example, mirror-polished aluminum (Al) having high ultraviolet light reflectivity, and the reflection surface 254 is coated with magnesium fluoride (MgF₂). The reflector 252 may be formed of a fluororesin material such as PTFE.

The reflector 252 has a bowl shape and has the reflection surface 254, which is a concave curve surface. Near the bottom portion of the reflector 252, a mounting hole 256 for arranging the light emitting device 42 is provided. The reflector 252 reflects a portion of ultraviolet light emitted by the light emitting device 42 and adjusts the direction of the ultraviolet light such that the light distribution angle of the ultraviolet light output from an opening 258 is 30 degrees or less. By applying the adjustment mechanism 250 according to the present exemplary variation to the above-stated irradiation apparatus, the same effects as those obtained in the above-described embodiment can be achieved.

Described above is an explanation based on the exemplary embodiments of the present invention. The invention is not limited to the above-mentioned embodiments, and various design modifications may be added. It will be obvious to those skilled in the art that such modifications are also within the scope of the present invention.

An explanation has been made considering that the irradiation apparatus 10 according to the above-stated embodiment is an apparatus for performing sterilization treatment by the irradiation of a fluid with ultraviolet light. In an exemplary variation, the present irradiation apparatus may be used for purifying treatment for decomposing organic substances included in a fluid by the irradiation with ultraviolet light.

An explanation has been made considering that the irradiation apparatus 10 according to the above-stated embodiment is an apparatus for performing sterilization treatment by the irradiation of a liquid such as water, as an example of the fluid, with ultraviolet light. In the exemplary variation, gas may be irradiated with ultraviolet light as the fluid.

### [DESCRIPTION OF THE REFERENCE NUMERALS]

10 irradiation apparatus, 20 straight pipe, 20a internal wall surface, 40 light source, 42 light emitting device, 50 adjustment mechanism

### [INDUSTRIAL APPLICABILITY]

According to the present invention, sterilization capability can be improved by increasing efficiency for irradiating the inside of a straight pipe with ultraviolet light.

## Claims

1. An irradiation apparatus comprising:
a straight pipe that is formed of polytetrafluoroethylene (PTFE); and
a light source that is arranged at an end portion of the straight pipe and that irradiates the inside of the straight pipe with ultraviolet light,
wherein the light source has a light emitting device that emits ultraviolet light and an adjustment mechanism that adjusts the direction of the ultraviolet light such that the ultraviolet light from the light emitting device becomes incident on the internal wall surface of the straight pipe at an incident angle of 75 degrees or more.

2. The irradiation apparatus according to claim 1, wherein the adjustment mechanism adjusts the direction of the ultraviolet light such that the light distribution angle of the ultraviolet light irradiating the inside of the straight pipe is 30 degrees or less.

3. The irradiation apparatus according to claim 1 or 2, wherein the length of the straight pipe is three or more times larger than the diameter of the straight pipe.

4. The irradiation apparatus according to any one of claims 1 to 3, wherein the light source irradiates a fluid flowing inside the straight pipe with ultraviolet light to perform sterilization treatment on the fluid.

5. A fluid sterilization method comprising:
irradiating a fluid flowing inside a straight pipe formed of polytetrafluoroethylene (PTFE) with ultraviolet light to perform sterilization treatment on the fluid,
wherein the ultraviolet light is irradiated in a direction where the ultraviolet light becomes incident on the internal wall surface of the straight pipe at an incident angle of 75 degrees or more.
